Europäisches Patentamt

European Patent Office

Office européen des brevets

(19)

(11) Veröffentlichungsnummer : **0 121 151**
**B1**

# (12) EUROPÄISCHE PATENTSCHRIFT

(45) Veröffentlichungstag der Patentschrift :
22.10.86

(21) Anmeldenummer : 84102598.4

(22) Anmeldetag : 09.03.84

(51) Int. Cl.⁴ : **A 61 B   6/06, G 03 B 42/02**

(54) **Röntgendiagnostikgerät.**

(30) Priorität : **25.03.83 DE 3310971**

(43) Veröffentlichungstag der Anmeldung :
**10.10.84 Patentblatt 84/41**

(45) Bekanntmachung des Hinweises auf die Patenterteilung : **22.10.86 Patentblatt 86/43**

(84) Benannte Vertragsstaaten :
**BE DE FR IT**

(56) Entgegenhaltungen :
**DE-A- 1 930 283
DE-A- 2 520 179
DE-A- 2 939 425
FR-A- 2 343 272
US-A- 3 502 872
US-A- 3 511 995
US-A- 4 152 604**

(73) Patentinhaber : **Siemens Aktiengesellschaft Berlin
und München
Wittelsbacherplatz 2
D-8000 München 2 (DE)**

(72) Erfinder : **Hüttenrauch, Gerd
Sudetenstrasse 14
D-8521 Uttenreuth (DE)**
Erfinder : **Seubert, Hans-Peter
Kübelohberg 5
D-8551 Heroldsbach (DE)**

EP 0 121 151 B1

## Beschreibung

Die Erfindung betrifft ein Röntgendiagnostikgerät mit einer Röntgenröhre, einer Primärstrahlenblende, einer Bildaufnahmeeinrichtung mit einem Belichtungsautomaten, der mehrere Meßfelder in einem Strahlendetektor sowie Schaltmittel zur Auswahl jeweils eines Meßfeldes aufweist, sowie mit Mitteln zum Einstellen des Film-Fokus-Abstandes.

Ein Röntgendiagnostikgerät dieser Art ergibt sich aus der DE-OS 19 30 283. Bei diesem bekannten Röntgendiagnostikgerät erfolgt die Wahl des jeweiligen Meßfeldes des Strahlendetektors durch eine Schaltvorrichtung in Abhängigkeit von der Stellung des Patientenlagerungstisches. Es ist auch möglich, Handschaltmittel vorzusehen, durch die das Meßfeld demgemäß von Hand ausgewählt werden kann.

In der Praxis kann es vorkommen, daß das von der Primärstrahlenblende auf dem Strahlendetektor eingeblendete Strahlenfeld nicht alle Meßfelder umfaßt. Wird nun ein Meßfeld gewählt, das außerhalb des eingeblendeten Strahlenfeldes liegt oder das nur teilweise von Röntgenstrahlung erfaßt wird, so treten Fehlbelichtungen auf.

Der Erfindung liegt die Aufgabe zugrunde, ein Röntgendiagnostikgerät der eingangs genannten Art so auszubilden, daß Fehlbelichtungen aufgrund fehlender oder nur teilweiser Durchstrahlung des gewählten Meßfeldes des Strahlendetektors vermieden werden.

Diese Aufgabe ist erfindungsgemäß dadurch gelöst, daß ein Geber für die Größe des jeweils auf dem Strahlendetektor eingeblendeten Strahlenfeldes vorhanden ist, der an einem Rechner angeschlossen ist, welcher das jeweils gewählte Meßfeld mit der Strahlenfeldgröße vergleicht und abschaltet, wenn es nicht voll innerhalb des Strahlenfeldes liegt. Bei dem erfindungsgemäßen Röntgendiagnostikgerät wird ein gewähltes Meßfeld nicht genutzt, wenn es nicht voll von Röntgenstrahlung getroffen wird, also nicht ganz innerhalb des eingeblendeten Strahlenfeldes liegt.

Eine besonders zweckmäßige Weiterbildung der Erfindung besteht darin, daß der Rechner so ausgebildet ist, daß er nach Abschaltung des gewählten Meßfeldes ein im Strahlenfeld liegendes Meßfeld einschaltet. Bei dieser Weiterbildung wird in jedem Fall eine Röntgenaufnahme angefertigt. Der Rechner zur Bestimmung der eingeblendeten Strahlenfeldgröße kann Signale erhalten, die der jeweiligen Stellung der Blendenplatten der Primärstrahlenblende und dem eingestellten Film-Fokus-Abstand entsprechen. Aus diesen Signalen kann er dann die auf dem Strahlendetektor eingeblendete Strahlenfeldgröße bestimmen.

Der Rechner kann auch den Parallaxenfehler bei seitlicher Einstrahlung, bei der der Zentralstrahl des Röntgenstrahlenbündels nicht senkrecht auf dem Strahlendetektor, der z. B. eine Ionisationskammer sein kann, auftrifft, berücksichtigen. Auch bei kleinen Kassettenformaten und dementsprechend kleinen Strahlendetektoren können bis zu einer gewissen Einblendgröße die seitlichen Meßfelder des Strahlendetektors für die Steuerung des Belichtungsautomaten zugelassen werden. Dies ist z. B. bei Magenuntersuchungen mit kleinen Kassetten vorteilhaft, bei denen das mittlere Meßfeld des Strahlendetektors durch das Kontrastmittel abgedeckt wird und nur die Wahl eines seitlichen Meßfeldes eine für die Diagnose erforderliche richtige Filmschwärzung ergibt.

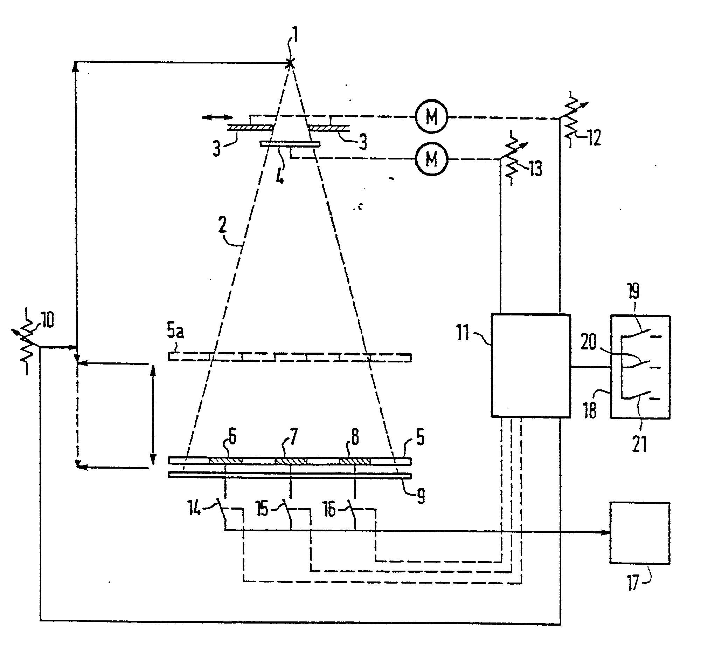

Die Erfindung ist nachfolgend anhand eines in der Zeichnung dargestellten Ausführungsbeispieles näher erläutert.

In der Zeichnung ist der Fokus 1 einer Röntgenröhre dargestellt, von dem ein Röntgenstrahlenbündel 2 ausgeht, das durch eine Primärstrahlenblende eingeblendet wird, von der zwei senkrecht zueinander verstellbare Blendenplattenpaare 3, 4 gezeichnet sind. Das Röntgenstrahlenbündel 2 durchsetzt eine Ionisationskammer 5 mit drei Meßfeldern 6, 7, 8 und trifft auf einer Röntgenfilmkassette 9 auf, die einen Röntgenfilm enthält, auf dem ein Röntgenbild erzeugt wird. Der Film-Fokus-Abstand ist durch nicht dargestellte Mittel einstellbar. Ein diesem Abstand entsprechendes Signal wird von einem Istwertgeber 10 einem Mikro-Computer 11 zugeführt. Der Mikro-Computer 11 erhält auch Signale von Istwertgebern 12 und 13 für die Länge und Breite des auf der Ionisationskammer 5 durch die Primärstrahlenblende 3, 4 eingeblendeten Strahlenfeldes.

Jeweils eines der Meßfelder 6, 7, 8 ist über eine Schaltvorrichtung 14, 15, 16 an einem Röntgenbelichtungsautomaten 17 anschaltbar, der die Abschaltung der Röntgenröhre bewirkt, wenn eine vorbestimmte Dosis auf das Meßfeld eingewirkt hat. Das jeweilige Meßfeld ist durch eine manuell betätigbare Schaltvorrichtung 18 auswählbar, die Schalter 19, 20, 21 aufweist. Wird beispielsweise der Schalter 19 betätigt, so schließt ein Relais den Schalter 14 und das Meßfeld 6 wird gewählt. Durch den Schalter 20 ist demgemäß das Meßfeld 7 und durch den Schalter 21 das Meßfeld 8 auswählbar.

Der Mikro-Computer 11 bestimmt aus seinen Eingangssignalen, d. h. aus der jeweiligen Stellung der Primärstrahlenblende 3, 4 und dem jeweiligen Film-Fokus-Abstand, die Größe des Strahlenfeldes auf der Ionisationskammer 5. Hierzu ist er entsprechend der Größe dieser Ionisationskammer 5 auch programmiert. Er vergleicht dann das durch die Schaltvorrichtung 18 jeweils manuell ausgewählte Meßfeld mit der Größe des Strahlenfeldes und entscheidet, ob es im eingeblendeten Strahlenfeld liegt. Ist dies der Fall, so verändert er die Meßfeldwahl nicht. Ist dies jedoch nicht der Fall, was bei der Wahl der Meßfelder 6 und 8 vorkommen kann, wie die gestrichelt gezeichnete Lage 5a der Ionisa-

tionskammer 5, d. h. Einstellung eines anderen Film-Fokus-Abstandes, zeigt, so schaltet er das gewählte Meßfeld ab und wählt das Meßfeld 7 durch Schließen des Schalters 15 automatisch aus, das dann auf jeden Fall in dem eingeblendeten Strahlenfeld liegt.

**Patentansprüche**

1. Röntgendiagnostikgerät mit einer Röntgenröhre (1), einer Primärstrahlenblende (3, 4), einer Bildaufnahmeeinrichtung (9) mit einem Belichtungsautomaten (5 bis 8, 14 bis 21), der mehrere Meßfelder (6, 7, 8) in einem Strahlendetektor (5) sowie Schaltmittel (14 bis 21) zur Auswahl jeweils eines Meßfeldes (6, 7, 8) aufweist, sowie mit Mitteln zum Einstellen des Film-Fokus-Abstandes, dadurch gekennzeichnet, daß ein Geber (10, 12, 13) für die Größe des jeweils auf dem Strahlendetektor (5) eingeblendeten Strahlenfeldes vorhanden ist, der an einem Rechner (11) angeschlossen ist, welcher das jeweils gewählte Meßfeld (6, 7, 8) mit der Strahlenfeldgröße vergleicht und abschaltet, wenn es nicht voll innerhalb des Strahlenfeldes liegt.

2. Röntgendiagnostikgerät nach Anspruch 1, dadurch gekennzeichnet, daß der Rechner (11) so ausgebildet ist, daß er nach Abschaltung des gewählten Meßfeldes (6, 7, 8) ein im Strahlenfeld liegendes Meßfeld (7) einschaltet.

3. Röntgendiagnostikgerät nach Anspruch 1 oder 2, dadurch gekennzeichnet, daß der Rechner (11) zur Bestimmung der eingeblendeten Strahlenfeldgröße Signale erhält, die der jeweiligen Stellung der Blendenplatten (3, 4) der Primärstrahlenblende und dem Film-Fokus-Abstand entsprechen.

**Claims**

1. X-ray diagnostic apparatus comprising an X-ray tube (1), a primary radiation diaphragm (3, 4), a picture recording device (9) with a radiation detector (5) that possesses a plurality of measuring fields (6, 7, 8) and switching means (14 to 21) for the respective selection of one measuring field (6, 7, 8) by an automatic exposure machine (5 to 8, 14 to 21), and means provided for adjusting the film-focus distance, characterised in that a generator (10, 12, 13) is provided for the radiation field, whose magnitude is respectively adjusted on the radiation detector (5), which generator is connected to a computer (11) which compares the respectively selected measuring field (6, 7, 8) to the radiation field magnitude and switches it off if it does not lie completely within the radiation field.

2. X-ray diagnostic apparatus as claimed in Claim 1, characterised in that the computer (11) is designed such that when the selected measuring field (6, 7, 8) has been switched off it switches on a magnetic field (7) which lies within the radiation field.

3. X-ray diagnostic apparatus as claimed in Claim 1 or 2, characterised in that the computer (11) receives signals which correspond to the respective position of the diaphragm plates (3, 4) of the primary radiation diaphragm and the film-focus distance in order to determine the adjusted magnitude of the radiation field.

**Revendications**

1. Appareil de radiodiagnostic comportant un tube (1) à rayons X, un diaphragme (3, 4) du rayonnement primaire, un dispositif d'enregistrement d'images (9) comportant un dispositif automatique d'exposition (5 à 8, 14 à 21), qui comporte plusieurs zones de mesure (6, 7, 8) dans un détecteur de rayonnement (5) ainsi que des moyens de commutation (14 à 21) servant à sélectionner respectivement une zone de mesure (6, 7, 8), ainsi que des moyens pour régler la distance film-foyer, caractérisé par le fait qu'il est prévu un générateur (10, 12, 13), qui indique l'étendue du champ de rayonnement formé respectivement par collimation sur le détecteur de rayonnement (5) et qui est raccordé à un calculateur (11) qui compare la zone de mesure (6, 7, 8) respectivement sélectionnée à l'étendue du champ de rayonnement et débranche cette zone de mesure lorsqu'elle n'est pas située complètement à l'intérieur du champ de rayonnement.

2. Appareil de radiodiagnostic selon la revendication 1, caractérisé par le fait que le calculateur (11) est agencé de telle sorte qu'après le débranchement de la zone de mesure (6, 7, 8) sélectionnée, il branche une zone de mesure (7) située dans le champ de rayonnement.

3. Appareil de radiodiagnostic suivant la revendication 1 ou 2, caractérisé par le fait que pour la détermination de l'étendue du champ de rayonnement collimaté, le calculateur (11) reçoit des signaux qui correspondent à la position respective des plaques collimatrices (3, 4) du diaphragme du rayonnement primaire et à la distance film-foyer.